Europäisches Patentamt

European Patent Office

Office européen des brevets

(11) Publication number: **0 092 962**
**A2**

## (12) EUROPEAN PATENT APPLICATION

(21) Application number: **83302206.4**

(22) Date of filing: **19.04.83**

(51) Int. Cl.³: **A 61 B 5/02**
**A 61 B 5/04**

(30) Priority: **28.04.82 JP 72078/82**

(43) Date of publication of application:
**02.11.83 Bulletin 83/44**

(84) Designated Contracting States:
**CH DE FR GB LI**

(71) Applicant: **KABUSHIKI KAISHA DAINI SEIKOSHA**
**31-1, 6-chome Kameido**
**Koto-ku Tokyo(JP)**

(72) Inventor: **Tabata, Junichi**
**Kabushiki Kaisha Daini Seikosha 6-31-1, Kameido**
**Koto-ku Tokyo(JP)**

(74) Representative: **Caro, William Egerton et al,**
**J. MILLER & CO. Lincoln House 296-302 High Holborn**
**London WC1V 7JH(GB)**

(54) Heartbeat rate indicator.

(57) A heartbeat rate indicator comprises a detection electrode (1) for receiving an electrocardiac potential signal, an amplifier circuit (3) for amplifying the electrocardiac potential signal, a filter circuit (4) for removing noise from the amplified electrocardiac potential signal, an an amplitude detection circuit (5) for detecting pulses having an amplitude greater than a predetermined amplitude. An absolute value circuit (8) produces an absolute value signal from the output signal of the filter circuit, the absolute value signal being fed to the amplitude detection circuit.

Fig.4.

- 1 -

"HEARTBEAT RATE INDICATOR"

This invention relates to heartbeat rate indicators or pulse detection circuits (pulsimeters).

Conventionally, heartbeat rate is measured by electrocardiography where a small electric potential produced by the heart prior to contraction is detected. It has been proposed to measure heartbeat rate by detecting an electrocardiac potential signal induced between two metal electrodes in contact with different parts of the human body. Theoretically it should be possible to provide a small size and long life heartbeat rate indicator because the electrocardiac potential signal can be detected with low power consumption (about 100 μW).

According to the present invention there is provided a heartbeat rate indicator comprising: a detection electrode for receiving an electrocardiac potential signal; an amplifier circuit for amplifying the electrocardiac potential signal; a filter circuit for removing noise from the amplified electrocardiac potential signal; and an amplitude detection circuit for detecting pulses having an amplitude greater than a predetermined amplitude, characterised by an absolute value circuit for producing an absolute value signal from the output signal of the filter circuit, the absolute value signal being fed to the amplitude detection circuit.

In the preferred embodiment the absolute value circuit includes a half-wave rectifier for half-wave rectifying the output signal from the filter circuit and an adder circuit for summing the half-wave rectified output signal from the half-wave rectifier with the output signal from the filter circuit.

The heartbeat rate indicator may include an operational amplifier between the absolute value circuit and the amplitude detection circuit.

The invention is illustrated, merely by way of example, in the accompanying drawings, in which:-

Figure 1 shows the waveform of a human heartbeat;

Figure 2 is a block diagram of a heartbeat rate indicator such as that disclosed in British Patent Application No. 8232441;

Figure 3 shows the waveform of a human heartbeat that cannot be detected accurately by the heartbeat rate indicator of Figure 2;

Figure 4 is a block diagram of one embodiment of a heartbeat rate indicator according to the present invention; and

Figure 5 illustrates the operation of an absolute value circuit of the heartbeat rate indicator of Figure 4.

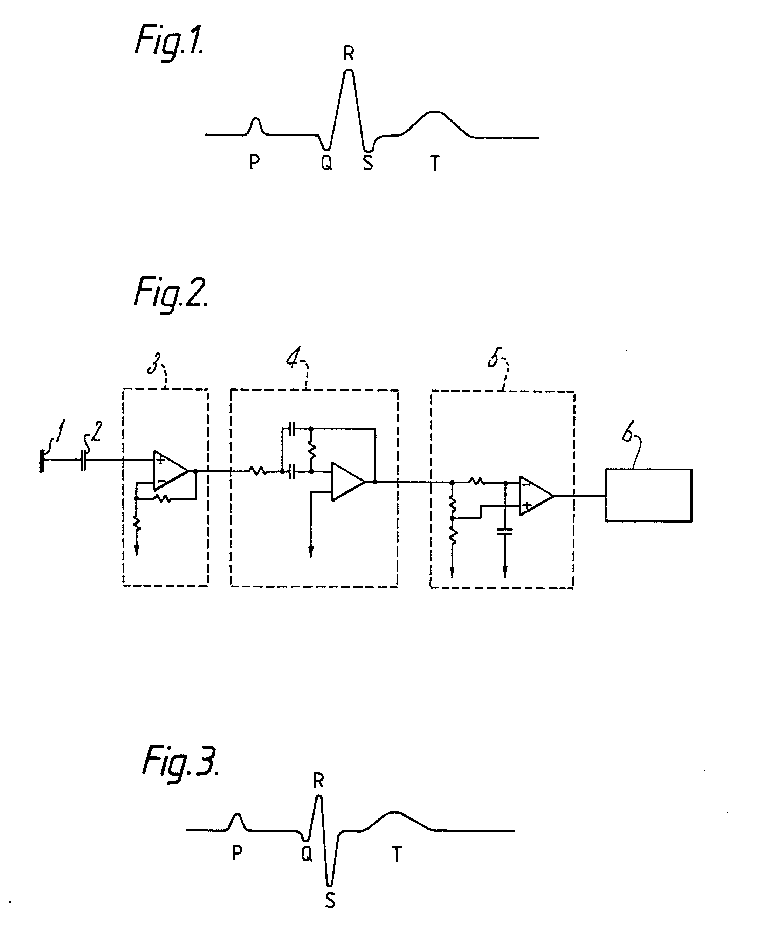

Figure 1 shows the waveform of a human heartbeat. Generally, an electrocardiac potential signal induced between the right limb of the human body and the left limb is composed of a P-wave, a Q-R-S-wave and T-wave for each contraction of the heart. The amplitude of the Q-R-S-wave is the largest of the three waves and ranges from about 0.2 mV peak-to-peak and 1.5 mV peak-to-peak. Normally heartbeat rate indicators detect the Q-R-S-wave.

On the top of the electrocardiac potential signal is superimposed noise at commercial frequencies induced in the surface of the human body. For heartbeat rate measurement it is necessary to eliminate relatively large amplitude noise and to detect the electrocardiac potential signal which is of relatively small amplitude.

Figure 2 illustrates a heartbeat rate indicator such as that disclosed in British Patent Application No. 8232441. A detection electrode 1 is of stainless steel. When a person measures his heartbeat rate, a portion of the body surface, for example, the skin of one hand (left hand) acts as ground potential and the skin of the other hand (right hand) for example the tip of a finger, contacts the detection electrode 1. Thus the heartbeat rate indicator receives an electrocardiac potential signal. The ground potential may be applied to a casing of the heartbeat rate indicator when it is worn on the wrist of the left hand.

The detection electrode 1 is connected to an amplifier circuit 3 *via* a capacitor 2 which acts as a DC elimination circuit. The amplifier circuit 3 is an operational amplifier with a plurality of resistors whose resistance ratio determines the amplification factor. The electrocardiac potential signal and noise amplified by the amplifier circuit are fed to a band-pass filter 4. The band-pass filter 4 consists of an operational amplifier, a resistor and two capacitors, the resistance ratio and the capacitance ratio determining the central frequency and the quality factor Q. Noise at commercial frequencies is eliminated by the band-pass filter 4 so that only the amplified electrocardiac potential signal appears at the output of the band-pass filter. The output signal from the band-pass filter 4 is fed to an AC amplitude detection circuit 5. The AC amplitude detection circuit 5 consists of a capacitor, a plurality of resistors and a voltage comparator and produces an output pulse signal only when the output signal from the band-pass filter has an amplitude greater than a predetermined value.

The pulse signal from the AC amplitude detection circuit 5 is fed to an arithmetic logic circuit 6 which calculates the number of pulses produced per minute. That is to say produces an indication of heartbeat rate as the number of heartbeats per minute.

Heartbeat rate can usually be determined with considerable accuracy using the heartbeat rate indicator of Figure 2. However, the heartbeat rate of some patients cannot be detected by it. Figure 3 shows the waveform of the heartbeat of one such patient. The waveform shown in Figure 3 differs from that of Figure 1 in that the amplitude change in the positive direction of the Q-R-S-wave is smaller than the amplitude change in the negative direction whereas the reverse is true for the waveform of Figure 1. An electrocardiac potential signal with the waveform shown in Figure 3 cannot be detected with accuracy using the heartbeat rate indicator of Figure 2 because the AC amplitude detection circuit 5 is designed to detect only a signal changing in the positive direction.

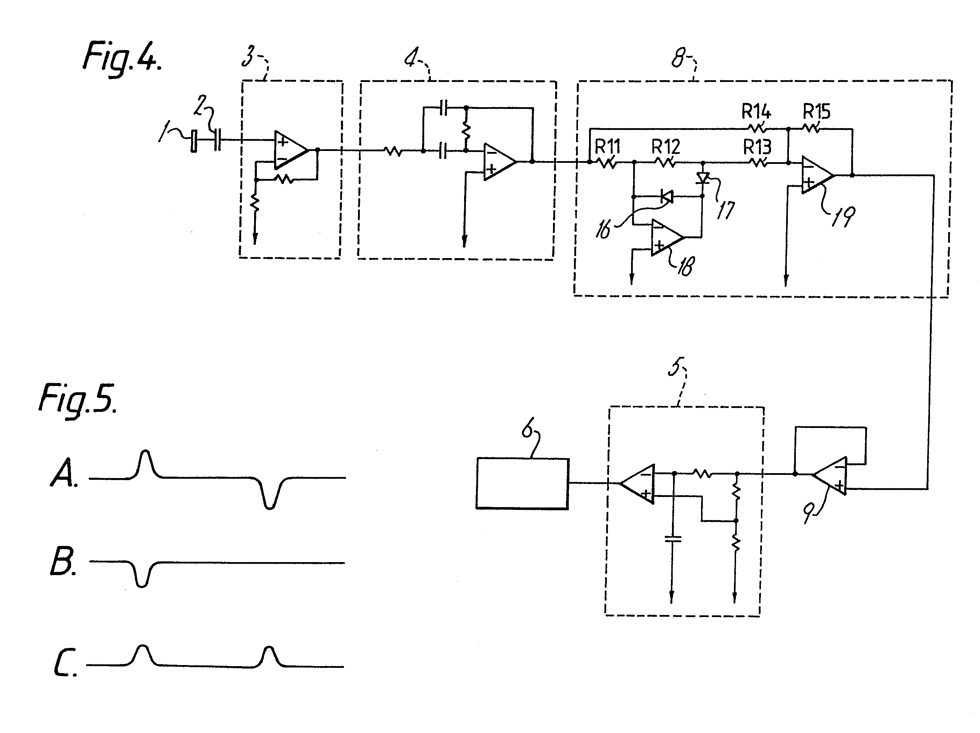

Figure 4 shows one embodiment of a heartbeat rate indicator according to the present invention. Like parts in Figures 2 and 4 have been designated by the same reference numerals and further description will be omitted. The heartbeart rate indicator of Figure 4 has an absolute value circuit 8, which is such that an electrocardiac potential signal which varies largely in the negative direction and which is hard to detect with the heartbeat rate indicator of Figure 2 is inverted and so is detectable.

The absolute value circuit 8 consists of resistors $R_{11}$, $R_{12}$, $R_{13}$, $R_{14}$, $R_{15}$, diodes 16, 17 and operational amplifiers 18, 19. An output signal from the band-pass filter 4 is connected to one side of the resistor $R_{11}$, the other side of which is connected to the inverting input of the operational amplifier 18. The non-inverting input of the operational amplifier 18 is connected to ground potential and its output terminal is connected to the anode of the diode 16 and the cathode of the diode 17. The cathode of the diode 16 is connected to the inverting input of the operational amplifier 18. One side of the resistor $R_{12}$ is connected to the inverting input of the operational amplifier 18 and the other side is connected to the anode of the diode 17.

The resistors $R_{11}$, $R_{12}$, the diodes 16, 17 and the operational amplifier 18 constitute a half-wave rectifier. The output and the input of the half-wave rectifier are summed by an adding circuit which produces a full-wave rectified signal at the output of the absolute value circuit 8. The adding circuit consists of the resistor $R_{13}$ one side of which is connected to the anode of the diode 17 and the other side of which is connected to the inverting input of the operational amplifier 19. One side of the resistor $R_{14}$ is connected to receive the output signal from the band-pass filter 4 fed to the absolute value circuit 8 and the other side of the

resistor $R_{14}$ is connected to the inverting input of the operational amplifier 19. One side of the resistor $R_{15}$ is connected to the inverting input of the operational amplifier 19 and the other side is connected to the output of the operational amplifier 19. The non-inverting input of the operational amplifier 19 is connected to ground potential and the output signal of the operational amplifier 19 is fed to the AC amplitude detection circuit 5 _via_ an operational amplifier 19.

The relationship between the resistance values of the five resistors of the absolute value circuit 8 are chosen to be:

$$R_{11} = R_{12} = R_{14} = R_{15} = 2R_{13}$$

Figure 5 illustrates the operation of the absolute value circuit 8. When a signal A having a waveform consisting of alternately occurring positive and negative pulses is applied to the input of the absolute value circuit, a half-wave rectified signal B is produced by the half-wave rectifier and a full wave rectified signal C is produced at the output. The waveform signal A is given merely for ease of explanation. In practice the absolute value circuit 8 receives an amplified and filter Q-R-S-wave from the band-pass filter 4.

The provision of the absolute value circuit enables the absolute value of the Q-R-S-wave to be detected regardless of whether it is positive going or negative going. Consequently heartbeat rate measurement can take place regardless of differences in polarity of the Q-R-S-waves of patients. Moreover, the pulse detection circuit is stable and highly precise.

CLAIMS

1.    A heartbeat rate indicator comprising:  a detection electrode
(1) for receiving an electrocardiac potential signal;  an amplifier
circuit (3) for amplifying the electrocardiac potential signal;
a filter circuit (4) for removing noise from the amplified
electrocardiac potential signal;  and an amplitude detection
circuit (5) for detecting pulses having an amplitude greater than
a predetermined amplitude, characterised by an absolute value
circuit (8) for producing an absolute value signal from the output
signal of the filter circuit, the absolute value signal being fed
to the amplitude detection circuit.

2.    A heartbeat rate indicator as claimed in claim 1 characterised
in that the absolute value circuit includes a half-wave rectifier
($R_{11}$, $R_{12}$, 16, 17, 18) for half-wave rectifying the output signal
from the filter circuit and an adder circuit ($R_{13}$, $R_{14}$, $R_{15}$, 19)
for summing the half-wave rectified output signal from the half-wave
rectifier with the output signal from the filter circuit.

3.    A heartbeat rate indicator as claimed in claim 1 or 2
characterised by an operational amplifier (9) between the absolute
value circuit and the amplitude detection circuit.

4.    In a pulse detecting circuit comprising:  a pulse detecting
portion consisting of an electrocardiac potential detection
electrode, an amplifying circuit, a filter circuit and an AC
amplitude detecting circuit;  and a signal proceessing circuit, said
pulse detecting circuit further comprising an absolute value circuit
at said pulse detecting portion.

**Fig.1.**

**Fig.2.**

**Fig.3.**

Fig.4.

Fig.5.

A.

B.

C.

R11  R12  R13  R14  R15

2/2

0092962